# EUROPEAN PATENT SPECIFICATION

(11) **EP 1 765 433 B1**
(45) Date of publication and mention of the grant of the patent: **18.05.2016**
(21) Application number: 05786088.4
(22) Date of filing: 20.06.2005
(51) Int. Cl.: A61M 1/36, A61M 1/34

(54) **PLASMA DETOXIFICATION AND VOLUME CONTROL SYSTEM**
PLASMAENTGIFTUNG UND VOLUMENSTEUERUNGSSYSTEM
SYSTEME DE DETOXIFICATION DU PLASMA ET DE REGULATION DU VOLUME SANGUIN

(30) Priority: 21.06.2004 US 581922 P
(43) Date of publication of application: 28.03.2007
(73) Proprietor: Hemolife Medical, Inc., San Clemente CA 92673 (US)
(72) Inventor: ROBERTS, Craig, P., Carlsbad, CA 92009 (US); LITZIE, Ken, Tustin, CA 92782 (US)
(74) Representative: Green, Mark Charles
(86) International application number: PCT/US2005/021901
(87) International publication number: WO 2006/002151

(56) References cited:
- EP-A- 0 787 500
- US-A- 5 679 260
- US-A1- 2004 228 829

## Description

### FIELD OF THE INVENTION

The present invention related to devices and associated methods for plasma detoxification. Specifically, the present invention relates to an extracorporeal system that uses an adsorption column containing activated charcoal and ionic and non-ionic resins in combination with a hemofilter to remove toxins associated with sepsis and renal failure, treat chronic metabolic acidosis and control patient plasma water without the use of a renal dialyzer or dialysate.

### BACKGROUND OF THE INVENTION

Despite advances in supportive care, septic shock remains a major cause of morbidity and mortality. In 1995, there were an estimated more than 750,000 cases of sepsis in the United States, of whom 383,000 (51.1%) received intensive care and an additional 130,000 (17.3%) were ventilated in an intermediate care unit or cared for in a coronary care unit (Angus DC et al. Epidemiology of severe sepsis in the United States: Analysis of incidence, outcome, and associated costs of care. Crit Care Med 2001; 29:1303-10). Mortality was more than 28% or 215,000 annually. The incidence and mortality of sepsis increase with age. Sepsis is the second leading cause of death in among patients in non-coronary intensive care units and the 10^{th} leading cause of death overall in the United States (Martin GS et al. The epidemiology of sepsis in the United States from 1979 through 2000. N Eng J Med 2003; 348:1546-54). Furthermore, sepsis substantially reduces the quality of life of those who survive. Care of patients with sepsis costs an average of $22,000 per patient resulting in an economic burden of nearly $17 billion annually in the United States alone.

Sepsis generally develops as a result of the host response to infection. The pathogenesis of sepsis represents a complex constellation of interconnected events. Sepsis is a form of severe systemic inflammation due to local and systemic effects of circulating pro-inflammatory mediators. With the identification of the systemic inflammatory response as a major component in the pathogenesis of the septic shock syndrome, much of the recent work has focused on modulating this response. This includes anti-endotoxin therapies in patients with Gram-negative sepsis and therapies to modulate the pro-inflammatory mediators produced in response to infection, such as TNF-alpha, platelet-activating factor and complement. High-flow hemofiltration has the potential advantage of clearing both endotoxin and pro-inflammatory mediators. The bacterial toxins generated by the infecting organisms trigger complex immunologic reactions. A large number of mediators, including tumor necrosis factor, leukotrienes, lipoxygenase, histamine, bradykinin, serotonin and interleukin-2, have been implicated in addition to endotoxin (the lipid fraction of the lipopolysaccharides released from the cell wall of gram-negative enteric bacilli). Presently, the only recommended therapeutic approach remains close microbiological surveillance. Prophylactic antibiotics and enteral decontamination have only a minor role: they may have an adverse effect by the selection of multiple resistant strains. (Ronco C et al. A pilot study of coupled plasma filtration with adsorption in septic shock. Crit Care Med 2002; 30:1250-55).

The scientific literature provides some interesting experimental alternatives for treating sepsis. See for example J.A. Kellum and M. K. Dishart. Effect of Hemofiltration Filter Adsorption on Circulating IL-6 Levels in Spectic Rats. Critical Care 2002, 6:429-433 (hereinafter "Kellum). Kellum discloses using a hydrogel-type membrane made from an acrylonitrile and sodium methallyl sulfonate copolymer to remove IL-6 from the blood of septic rats. Reduction in overall IL-6 levels was noted, however, the filter used has a limited absorption profile and not all sepsis-associated cytokines are removed.

However, many cytokines and other toxins are bound to the blood protein albumin. Conventional dialysis membranes do not remove substantial quantities of these protein-bound toxins from the blood because protein-impermeable membranes are generally used. Consequently, other extracorporeal circuits such as continuous renal replacement therapies (CRRT), coupled plasma filtration adsorption (CPFA) and continuous veno-venous hemodiafiltration (CWHDF) may help minimize cell-associated cytokine concentrations is the blood of septic patents. See for example C. Tetta et al. Endotoxin and Cytokine Removal in Sepsis. Ther. Apher. 2002. 6:109-115. (hereinafter "Tetta"). Tetta concluded that CPFA may be preferable to CRRT and CWHDF for treating septic patents, but that much clinical research was need to prove efficacy. These more invasive detoxification methods enable higher clearance of protein-bound toxins due to direct contact between the sorbent and the albumin/toxin-complex.

Continuous veno-venous hemofiltration (CWH) was designed as a renal replacement therapy for patients with acute renal failure. In hemofiltration the blood is forced through a semipermeable membrane and water and small molecules are filtered out of the blood. Hemofiltration is slower and less physiologically disturbing than hemodialysis, it is often chosen over intermittent hemodialysis when blood pressure instability is a problem and CWH is generally more efficient than peritoneal dialysis. In some intensive care units the use of CWH is increasing, as appreciation builds for its utility in the management of non-oliguric patients, in particular those with multiple organ dysfunction or failure, when their treatment includes very large amounts of intravenous fluids. And finally, experimental work is focusing on the possible role of CVVH as an adjunct in the treatment of the sepsis syndrome.

Healthy kidneys regulate the body's internal environment of water and salts and excrete the end products of the body's metabolic activities and excess water (urine). They also produce and release into the bloodstream hormones that regulate vital functions including blood pressure, red blood cell production, and calcium and phosphorus metabolism. Impaired kidney function may affect any or all of these processes and may be due to problems in the kidney, a disease in other organs, or caused by normal, age-related processes. It may be acute or chronic and either minor or life threatening. All of these distinctions are important determinants of prognosis and appropriate treatment. When a person's loss of kidney function is so severe as to be incompatible with life, the person is said to be in renal failure.

Acute Renal Failure (ARF) is a syndrome with multiple causes; its associated consequences affect all organ systems. Defined as a sudden loss of renal function (over several hours to several days), ARF results in derangements in extracellular fluid balance, acid base, electrolytes and divalent cation regulation. An increased serum creatinine concentration, accumulation of other nitrogen-based waste products, and often a decline in urinary output are the hallmarks of ARF.

Although many advances in organ support technologies have occurred during the past two decades, the absolute mortality rates for ARF acquired in the hospital and in the intensive care unit are approximately 45% and 70%, respectively (Thadhani R et al., Acute renal failure, N Engl J Med. 334:1448-60, 1996). However the demographics of ARF have changed, with patients generally being older and having a higher acuity of illness. Fortunately, renal function recovery (ability to discontinue dialysis) in the past 20 years has remained greater than 50-75% in survivors of ARF.

More than 20 definitions of ARF have been published to date. Despite the difficulty in defining the syndrome, ARF occurs in approximately 1% of hospitalized patients, in as many as 20% of patients treated in ICUs and as many as 4-15% of patients after cardiovascular surgery. Approximately 30% of patients who experience ARF will require renal replacement therapy (peritoneal dialysis, intermittent hemodialysis or continuous hemofiltration). Community-acquired ARF occurs in approximately 209 patients per one million population, and the frequency of this syndrome is increasing in hospitalized patients (Liano F et al., Epidemiology of acute renal failure: a prospective, multicenter, community-based study, Kidney Int. 50:811-8, 1996).

More than 50 identified pathophysiologic pathways are responsible for ARF. Traditionally, the evaluation of ARF has focused on the determination of whether the cause of renal failure is prerenal (60% of community-acquired ARF, a condition resulting in decreased "effective renal perfusion"), postrenal (5-15% of community-acquired ARF, an obstruction to urinary outflow) or intrinsic renal (due to pathophysiologic derangements in the renal tubules, interstitium, vasculature or glomeruli; includes acute tubular necrosis, the most common cause of ARF in hospitalized patients) (Albright RC Jr. et al., Acute renal failure: a practical update, Mayo Clin Proc. 76:67-74, 2001).

Therapy to correct the pathophysiological impairments of ARF can be either nondialytic or dialytic in nature. Nondialytic therapy is focused on insuring that renal perfusion is maximized and correcting impairments. A variety of growth factors, hormones and drugs are currently under evaluation as nondialytic therapy for ARF. Dialytic therapy consists of peritoneal dialysis, intermittent hemodialysis or continuous hemofiltration.

Renal dialysis is an artificial method of maintaining the chemical balance of the blood when the kidneys have failed. The term dialysis refers to the process in which soluble waste products are separated from the blood using a semipermeable membrane. The blood is cleansed of impurities by cycling the blood through a machine containing a hemodialyzer membrane. On the other side of the membrane is a solution comprised of specific components that extract the impurities from the patient's blood. This solution is called the dialysate. Blood is both removed and returned to the patient via catheters. The effectiveness of dialysis depends on both its duration and efficiency.

Peritoneal dialysis, in contrast to hemodialysis, which cleanses the blood outside the body, works inside the body using the peritoneal membrane as the semipermeable barrier through which the blood can be filtered. The dialysate is infused directly into the patient's peritoneal cavity through a catheter; the cavity is used as a reservoir for the dialysate. Toxins in the blood filter through the peritoneal membrane into the cleansing solution, which is then withdrawn from the body through the same catheter and discarded. This procedure can be self-administered by patients several times a day.

Hemodialysis allows the extracorporeal removal of water and solutes from the blood by diffusion across a concentration gradient. Blood is pumped along one side of a semi-permeable membrane and a crystalloid solution is pumped in the opposite direction on the other side of the membrane. Solutes of very small molecular weight diffuse across the membrane in an attempt to equilibrate their concentrations. The pore size in the semi-permeable membrane determines its utility in ultrafiltration. Ultrafiltration membranes that are utilized in hemofilters allow the passage of molecules with a molecular weight of less than 20,000 Daltons. Thus ions and small chemicals present in plasma are filtered freely, including sodium, potassium, phosphate, bicarbonate, glucose and ammonia. So are larger soluble endogenous substances such as myoglobin, insulin, and interleukins, and certain exogenous substances circulating in plasma, including medications (vancomycin, heparin) and toxins (endotoxin, pesticides). Molecules that are bound to plasma proteins would not be filtered effectively by an ultrafiltration membrane.

Many side effects of hemodialysis are caused by rapid changes in the body's water and electrolyte balance during dialysis. These include muscle cramps, hypotension, complement activation and leukopenia. In addition patients undergoing peritoneal dialysis run the risk of serious peritoneal infections, some of which can progress to septic shock.

Extracorporeal circuits are well known in the prior art. However, the known extracorporeal circuits are used primarily as artificial kidneys and perfusion devices. Perfusion devices, sometime referred to as extracorporeal membrane oxygenation (ECMO) devices, are primarily used to provide circulatory assistance after open heart surgery. Kidney diafiltration, dialysis and pure hemofiltration are processes used to replace the function of the failing or diseased kidney. These devices principally rely on semi-permeable membrane technology and the principles of osmotic diffusion to remove proteins, salts and urea from the blood. Additionally, kidney dialysis can be combined with ultrafiltration to remove excess fluid from the blood or be combined with substitution infusion fluid to replace fluids and salts lost in the hemodiafiltration process. However, extracorporeal circuits used to augment and/or replace diseased kidneys are not designed to remove the complex biological toxins the liver is responsible for.

United States Patent Number 6,186,146 B1 (hereinafter "the '146 patent") issued February 13, 2001 to Glickman discloses an extracorporeal circuit having a filter device incorporated therein. Specifically, the '146 patent describes a treatment for cancer where cytotoxic drugs and biological agents are infused directly into a diseased organ. The patent's blood, leaving the treated organ, is diverted via an extracorporeal circuit wherein the cytotoxic and/or biological agent is removed from the blood via an inline filter before reaching the general circulation. No details as to the filter's composition are provided. However, the simple extracorporeal circuit disclosed in the '146 patent is intended to remove a defined concentration of a specific known chemotherapeutic and/or biological agent. It is not intended as a general replacement for a diseased organ. Moreover, no details are provided as to how one of ordinary skill in the art would use the disclosed device to remove other biological toxins.

US 2004/0228829 published on 18 November 2004 and filed on 23 February 2004 by Roberts et al describes a device for removing toxins from the blood of patients comprising activated charcoal and at least one non-ionic resinfor use in an extra-corporeal circuit.

US 5679260 published on 21 October 2007 to Boos et al discloses a process for the removal of tumour necrosis factor and / or bacterial lipopolysaccharides from bodily fluids such as blood in an extracorporeal perfusion system, including precipitation and filtration.

EP 0787500 published on 6 August 1997 to Bellco S.P.A. discloses an extracorporeal device for extracorporeal removal of toxins, in particular cytokines, from blood, including ultrafiltration or plasmafiltration, followed by an adsorption columnusing uncoated activated carbon and / or hydrophobic or ion-exchange polystyrene resins.

Consequently, there remains a need for extracorporeal devices and methods that can be used to safely remove toxins from plasma in patients suffering from sepsis. Additionally, there remains a recognized need for extracorporeal devices and methods useful for removing toxins and balancing plasma water in patients with acute renal disease.

### SUMMARY OF THE INVENTION

The present invention relates to an extracorporeal plasma detoxification system that can remove toxins associated with and resulting from sepsis and renal failure and correct electrolyte imbalance, treat chronic metabolic acidosis and control patient plasma water in patients in renal failure without the use of a renal dialyzer or dialysate. The present invention comprises an adsorptive toxin removal device with a hemofilter to effectively detoxify human plasma and balance blood volume in patients suffering from sepsis and renal failure. The invention is defined as claimed in Claim 1.

### BRIEF DESCRIPTION OF THE DRAWINGS

Figure 1 is a schematic diagram of an embodiment of the extracorporeal plasma detoxification system of the present invention.
Figure 2 depicts one embodiment of the adsorptive toxin removal device.
Figure 3 a-c graphically depicts the efficacy of the adsorptive toxin removal device in decreasing initial blood levels of bilirubin, urea nitrogen and creatinine.
Figure 4 graphically depicts the efficacy of the adsorptive toxin removal device in decreasing blood acetaminophen concentration.
Figure 5 a-f graphically depicts testing demonstrating that inclusion of the adsorptive toxin removal device into an extracorporeal circuit did riot result in evidence of hemodynamic instability (FIG. 5a), hemolysis (FIG. 5b), thrombocytopenia (FIG. 5c), leucopenia (FIG. 5d), or nonspecific loss of fibrinogen (FIG. 5e) or albumin (FIG. 5f) (Animals identified as C-J; ET=End of Treatment values)

### DEFINITION OF TERMS

The following definition of terms is provided as a helpful reference for the reader. The terms used in this patent have specific meanings as they related to the present invention. Every effort has been made to use terms according to their ordinary and common meaning. However, where a discrepancy exists between the common ordinary meaning and the following definitions, these definitions supercede common usage.

Absorbent: As used herein "absorbent" refers to a medium such as activated carbon, an ionic or a non-ionic resin that retains a biologically active organic molecule or inorganic salt. Generally, absorbent refers to something that absorbs. Absorption is the taking in by chemical or molecular attraction similar to how water is taken in and held by a sponge.

Adsorbent: As used herein "adsorbent" refers to a medium such as activated carbon, an ionic or a non-ionic resin that retains a biologically active organic molecule or inorganic salt. Generally, adsorbent refers to something that adsorbs. Adsorption is the taking up and holding by chemical attraction to the surface of a solid substance similar to how a cloth may adsorb large dye molecules by holding them on the surface of the fibers by chemical attraction.

Exchange Resin: As used herein "exchange resin" generally refers to the ionic or non-ionic exchange resin component of the present invention. Furthermore it is understood that term exchange resin may be used collectively to refer to both ion exchange resins and non-ionic exchange resins in those embodiments where an exchange resin is added to the extracorporeal circuit in combination with the adsorptive toxin removal device of the present invention.

Toxin removal device: As used here "toxin removal device" refers to one or more cartridges or containers that contain one or more adsorbents or absorbents capable of removing organic molecules and/or inorganic salts from plasma or other biological fluids. The inventors believe that most ionic and non-ionic resins and activated charcoal act as adsorbents by attaching to their surface and retaining thereon organic molecules and inorganic salts. However, the present inventors do not wish to be bound by this theory. Therefore, for the purposes of this invention, the term "toxin removal device" will include materials that either adsorb or absorb molecules from the blood and/or plasma of patients.

Moreover, the term "toxin removal device" can mean a single unitary device wherein one or more toxin removing compounds are contained therein, either mixed or physically separated. However, the term "toxin removal device" can also refer to a plurality of discrete unitary devices each containing one or more separate toxin removal compositions. The discrete devices may be connected in series depending on the device design and application.

Toxin: As used herein "toxin" refers to any organic or inorganic compound that when present in a patient's blood above a tolerable threshold causes an adverse effect on the patient. Representative examples include, but are not limited to cytokines including interleukins, interferons, tumor necrosis factor alpha, or gamma, soluble proteins, bilirubin, creatinine, amino acids, nucleic acids, bacterial toxins including endotoxins, exotoxins, lipopolysacccharides, cellular enzymes, bacterial cell wall components and pharmaceuticals such as acetaminophen.

### DETAILED DESCRIPTION OF THE INVENTION

The present invention describes an extracorporeal plasma detoxification system that can remove toxins associated with sepsis and renal failure and correct electrolyte imbalance, treat chronic metabolic acidosis and control patient plasma water in patients in renal failure without the use of a renal dialyzer or dialysate. The present invention combines an adsorptive toxin removal device with a hemofilter to effectively detoxify human plasma and balance blood volume in patients suffering from sepsis and renal failure.

The major danger associated with sepsis is septic shock caused by the release of endotoxin associated with bacterial cell walls. These toxins cause inflammatory responses by over-exciting the immune system. The immune response deals well with relatively minor invasions but such a massive overload can cause major shock in which the blood pressure falls dramatically. However, once sepsis has set in, treatments which kill the bacteria make the problem worse by causing the release of more bacterial endotoxin from the dying bacteria. Therefore sepsis is a life-threatening condition that until now was extremely difficult to treat or control.

Acute renal failure is defined as the sudden loss of kidney function (over several hours to days) resulting in derangement of extracellular fluid balance, acid base, electrolytes and divalent cation regulation. Additionally, increased serum creatinine concentration, accumulation of other nitrogen-based waste products and often a decline in urinary output are hallmarks of acute renal failure. Renal dialysis is required when the pathophysiologic derangements in electrolytes, fluid balance and waste levels reach undesirable levels. However, current renal dialysis technology does not address the needs of the patient in acute renal failure.

Consequently, the present inventors have developed an extracorporeal system useful for removing toxins from the plasma of patients suffering from sepsis. Additionally, the present invention will also remove toxins from the plasma, and plasma water as well as balance electrolytes and water in the plasma of patients suffering from renal failure.

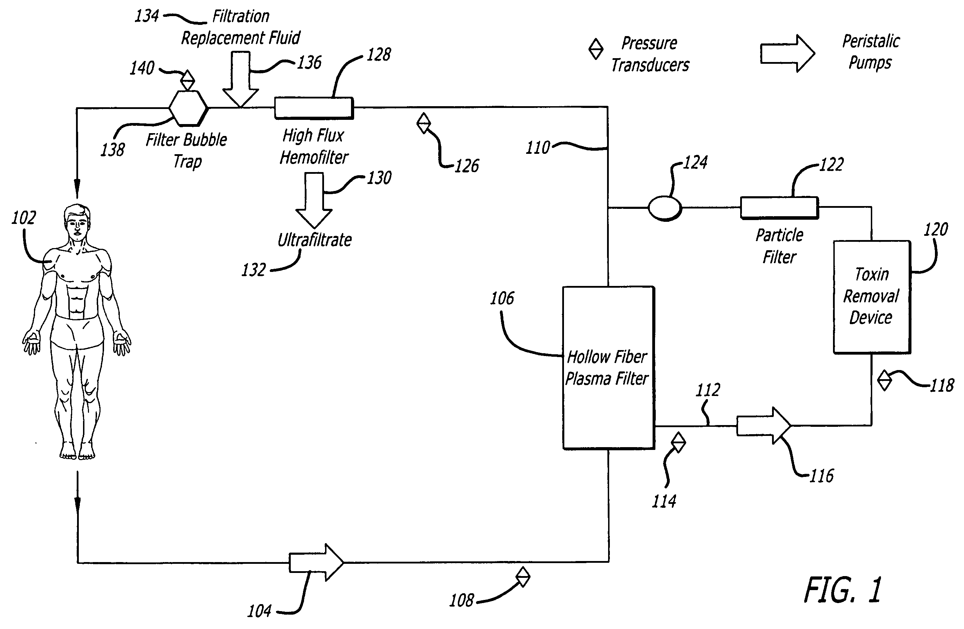

One embodiment of the extracorporeal system of the present invention will be described generally with reference to FIG. 1. In FIG. 1 blood is aspirated from a patient **102** in need of plasma detoxification or plasma water balancing. Blood is aspirated from the large vein of a patient via one lumen of a conventional dual lumen catheter connected to a peristaltic pump **104** and directed into a plasma filter **106** where the blood cells are separated from the plasma fraction of the blood. A pressure transducer **108** is provided between peristaltic pump **104** and hollow fiber plasma filter **106** to assess the flow/pressure characteristics of the resistance components. In one embodiment of the present invention a suitable plasma separation filter is a hollow fiber filter having a total surface area of 1 square meter and a 0.45 µM cutoff provided by Minntech, Inc. (Minneapolis, MN). The separated blood leaves the hollow fiber plasma filter **106** and can continue in one of two pathways. Blood cells are returned to the patient via pathway **110** and the separated plasma enters pathway **112.** At pathway **112** the plasma is pulled across the semi-permeable membrane of the plasma filter **106** by peristaltic pump **116** and propelled through adsorption column **120,** the toxin removal device of the present invention, which contains a mixture of adsorbent materials. The flow of plasma through pathway **112** is monitored by pressure transducers **114** and **118.** The purpose of the adsorptive toxin removal device is to remove both protein-bound and soluble toxins. Leaving adsorbent column **120,** the plasma flows through a particle filter **122** before being recombined with the patient's blood cells from pathway **110** at a connection between plasma filter **106** and hemofilter **128.** In addition, an optional heat exchanger **124** in the extracorporeal circuit can assist in the maintenance of patient temperature.

In one embodiment of the present invention, blood leaving the plasma filter **106** combines with plasma leaving particle filter **122** then through pressure transducer **126** and enters a high flux (0.3 m², 55,000 Dalton fiber membrane permeability) hemofilter **128.** The high flux hemofilter **128** allows for both the exchange of plasma water using a technique commonly referred to as zero balance ultrafiltration (ZBUF) and the removal of excess patient plasma water with a technique commonly referred to as continuous veno-venous hemofiltration (CWH). The addition of a balanced electrolyte replacement solution (filtration replacement fluid) **134** utilized in the ZBUF technique is controlled by peristaltic pump **136.** The removal of plasma water (ultrafiltrate) **132** in the CWH technique is also controlled by roller pump **130.**

In the final step, extracorporeal air is removed by bubble trap **138** equipped with a pressure transducer **140** in the circuit before returning to patient **102.**

The adsorptive toxin removal device is comprised of biologically active materials that adsorb (or absorb, see discussion *supra*) blood plasma-borne toxins that accumulate due to sepsis or diminished kidney function. The toxin removal device of the present invention may contain one or more material selected from the group consisting of activated charcoal and ion exchange resins. Essentially, ion exchange resins are classified as cation exchangers, which have positively charged mobile ions available for exchange and anion exchangers, whose exchangeable ions are negatively charged. Non-ionic exchange resins bind macromolecules via intermolecular forces, also referred to as van der Waal's forces, the weak attractive forces that hold non-polar molecules together (or non-polar regions of molecules having polar groups).

Both anion and cation resins are produced from the same basic organic polymers. However, they differ in the ionizable group attached to the hydrocarbon network. It is this functional group that determines the chemical behavior of the resin. Ionic exchange resins can be broadly classified as strong or weak acid cation exchangers or strong or weak base anion exchangers. In an ion exchange process, cations or anions in a liquid solution (usually aqueous) replace dissimilar and displaceable ions of the same charge contained in the ion exchange resin.

Non-ionic exchange resins are particular advantageous when used in accordance with the teachings of the present invention because they are less prone to bind (and thus removed from the blood) essential cations and anions such as, but not limited to, calcium, magnesium, sodium, potassium, chloride, carbonates, and other ionic species.

Specific non-limiting examples of non-ionic exchange resins suitable for use with the present invention include Amberlite™ XAD-7 HP and Amberchrom™ CG300-C. Amberlite™ is a group of polymeric synthetic resins made by the Rohm and Haas Company having a North American headquarters at 100 Independence Mall West Philadelphia, PA 19106-2399. Amberliten™ resins are available worldwide thorough a distributor network know to those skilled in the art. In one specific embodiment the present inventors have used Amberlite™ XAD-7 HP which is an aliphatic ester resin having an average surface area of approximately 500 m²/g and an average pore size of approximately 450 Angstroms and a mean diameter of approximately 560 µm.

Amberchrome™ CG300-G is a synthetic non-ionic exchange resin, also manufactured by Rohm and Haas, made from polystyrene divinyl benzene having an average surface area of approximately 700 m²/g with an average pore size of 300 Angstroms; mean particle diameter ranges from approximately 35 µM to approximately 120 µM.

However, whether the non-ionic exchange resins are used individually or in combination is not meant to be limiting, persons having ordinary skill in the art can easily select the exchange resin(s) best suited for a particular application. The factors that should be considered when selecting an appropriate exchange resin include the size, shape and charge of the molecule. Toxic molecules are general small and possess a few areas of high electron density but are known to possess carboxylic acid and amine residues that are easily polarizable and capable of hydrogen bonding. Larger macromolecules including cytokines, lymphokines and other toxic proteins have strong intermolecular forces suitable for removal using non-ionic resins that depend on van der Waal's forces to attract and bind molecules.

The activated carbon component of the toxin removal device comprises elementary carbon in a graphite-like structure. It can be produced by heat treatment, or "activation," of raw materials such as wood, coal, peat and coconuts. During the activation process, the unique internal pore structure is created, and it is this pore structure which provides activated carbon its outstanding adsorptive properties. Activated carbon is a carbonaceous adsorbent with a high internal porosity, and hence a large internal surface area. Commercial activated carbon grades have an internal surface area of 500 up to 1500 m²/g. Two representative, non-limiting, examples of commercially available activated carbon include Carbomix™, available from Norit, Nederland B.V. Headoffice P.O. Box 105 3800 AC Amersfoort, The Netherlands and Ultracarbon™ available through Merck & Co., Inc., Whitehouse Station, NJ.

In one embodiment, a standard dual lumen hemodialysis catheter is required for performing treatments. Blood is removed through the arterial line of the hemodialysis catheter by the action of the continuous roller pump **104** at a relatively low blood flow rate of approximately 125 mL/min. The toxin-containing blood then enters a plasma filtration step **106.** The plasma filter provides the continuous plasma filtration mode to generate plasma. This ensures that low, middle, and large molecular weight toxins are able to come into direct contact with the adsorbent filter while the cellular components of blood such as RBCs, platelets and leukocytes remain separate to avoid the drawbacks of direct hemoperfusion columns. Previous hemoperfusion columns were placed directly in the blood path, which allowed for activation and sequestration of platelets. The plasma filtrate that is generated is pumped by a second roller pump **116** at a rate of approximately 25 mL/min and passed through the toxin removing adsorbent column **120.** In an embodiment of the present invention, the adsorptive toxin removal device contains activated uncoated coconut shell (carbon granules) charcoal, and the non-ionic resins Amberlite™ XAD-7HP and Amberchrom™ GC300C.

In an embodiment of the present invention, the adsorptive toxin removal device is coated with albumin during a priming process during which albumin in the priming solution coats the adsorbent column **120** further increasing its biocompatibility. Previously activated carbon adsorbents were coated with cellulose to prevent platelet activation and to limit binding of beneficial plasma proteins. Cellulose, however, has the undesirable effect of decreasing total binding efficiency. The present inventors have determined that pre-treating the adsorbent media with albumin during priming causes the adsorbent to be more biocompatible and decreases binding of beneficial plasma proteins. In yet another embodiment of the present invention, rre-treating the adsorbent media with albumin may also occur during the manufacturing process.

In an embodiment, the filtration replacement fluid 134 can be customized to the specific electrolyte and fluid needs of the patient. In CVVH, the CVVH system can rapidly remake the extracellular fluid in the image of the filtration replacement fluid. Higher concentrations of bicarbonate are occasionally used in patients with severe metabolic acidosis, but overcorrection can develop rapidly resulting in metabolic alkalosis. Precise fluid balance is one of the beneficial features of CVVH. The volume of filtration replacement fluid is adjusted each hour to yield the desired fluid balance. All fluids removed from the patient (ultrafiltrate, urine, gastric drainage, etc.) and all fluids administered to the patient except for the filtration replacement fluid are totaled each hour. The desired fluid balance (e.g. removal of 1000 mL/hr) is determined from clinical considerations.

There are significant advantages to the present invention over currently used renal dialysis techniques. First, the use of an adsorption column allows for the targeted removal of both protein-bound and soluble toxins associated with sepsis and renal failure that cannot be removed or removed efficiently, using standard renal dialysis. Secondly, the use of combined ZBUF and CVVH facilitates the removal of waste materials and the stabilization of electrolytes as well as the simultaneous controlled removal of excess plasma water. Additionally, the composition of the ZBUF replacement fluid can be customized to meet patient physiologic requirements.

### EXAMPLES

The following examples are not intended as limitations. Rather they demonstrate illustrative embodiments of the present invention.

### Example 1 In Vitro Clearance Capabilities of the Adsorptive Toxin Removal Device

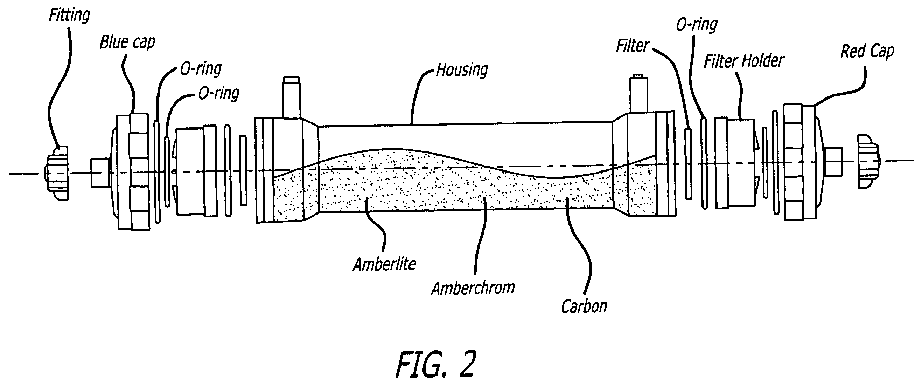

To demonstrate the efficacy of the adsorptive toxin removal device of the present invention (FIG. 2), human plasma spiked with bilirubin (20 mg/dL), urea nitrogen (50 mg/dL), and creatinine (5 mg/dL) was circulated through a closed system with separate columns containing activated charcoal, Amberlite™ XAD-7HP, and Amberchrom™ GC 300C (referred to herein individually as "sorbants") for 6 hours. The sorbants demonstrated varying effectiveness in clearing bilirubin, urea nitrogen and creatinine: activated charcoal (36 gm) decreased the levels of bilirubin by 49.5%, urea nitrogen 24.7%, and creatinine 97.9% of baseline values; Amberlite™ XAD-7HP (31 gm) decreased the levels of bilirubin by 34.6%, urea nitrogen 11.2%, and creatinine 9.0% of baseline values; Amberchrom™ GC300C decreased the levels of bilirubin by 95.7%, urea nitrogen 11.2%, and creatinine 10.1% of baseline values. Associated with these clearances was a modest 15-20% decrease in plasma albumin and total protein concentrations.

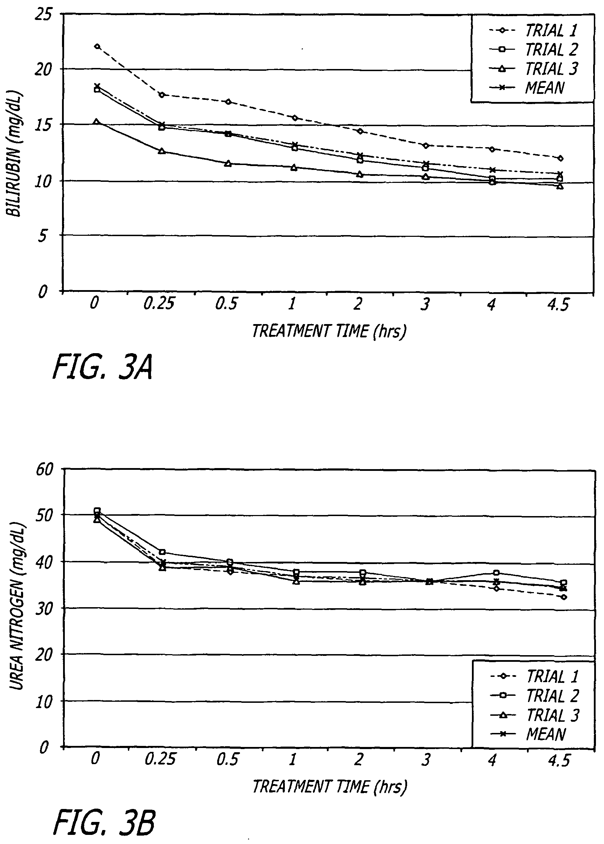

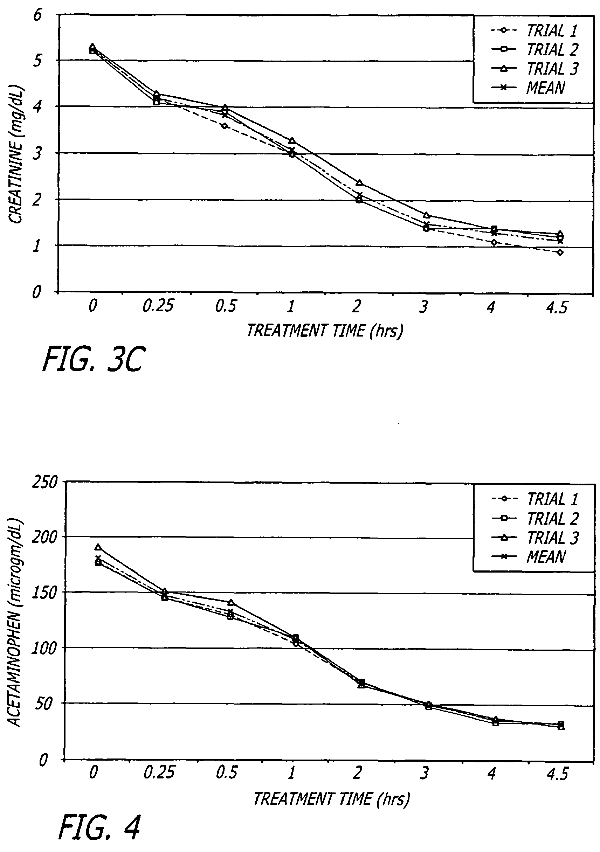

A separate series of *in vitro* experiments was carried out utilizing the adsorptive toxin removal device of the present invention containing 100 gm activated uncoated coconut shell granule charcoal, 35gm Amberlite™ XAD-7HP, and 35gm Amberchrom™ GC300C (dry weights) (the HLM-100 adsorptive column). Heparinized human plasma spiked with bilirubin, urea nitrogen, and creatinine at approximate initial concentrations of 20 mg/dL, 50 mg/dL, and 5 mg/dL, respectively. The adsorptive toxin removal device of the present invention decreased initial bilirubin, urea nitrogen, and creatinine levels by 41.4%, 30.7%, 78.3% respectively (see Table 1 and FIG. 3). In addition to these endogenous toxins, acetaminophen was added to the plasma at an initial concentration of approximately 175-200 micrograms/mL. The adsorptive toxin removal device decreased the initial acetaminophen concentration by 82.4% (see FIG. 4). There was a modest 10-15% decrease in total protein and albumin, in addition to a 25-30% fibrinogen decline with this embodiment of the present invention.

**Table 1. In Vitro Investigation of the HLM-100 Adsorptive Toxin Removal Device**

| Toxins | Percent Decrease in Initial Toxin Levels (Percent ± SD) |
|---|---|
| Endogenous Toxins | |
| bilirubin | 41.4 ± 4.2 |
| Urea Nitrogen | 30.7 ± 2.9 |
| creatinine | 78.3 ± 3.8 |
| Exogenous Toxin | |
| Acetaminophen | 82.4 ± 1.3 |

This *in vitro* data confirms the ability of the adsorptive toxin removal device to effectively remove toxins associated with acute liver failure and acute-on-chronic liver failure. The clearance of bilirubin also indicates the clearance of bile acids, which are toxic to hepatocytes, impair CNS function and the immune response to infection. The removal of acetaminophen confirms the ability of the adsorptive toxin removal device to remove exogenous toxins.

### Example 2 Safety Testing of the Adsorptive Toxin Removal Device

The safety of the adsorptive toxin removal device was also investigated in a canine extracorporeal circulation model involving eight approximately 55 pound mongrel dogs. The purpose of the safety testing was to demonstrate that the adsorptive toxin removal device does not remove beneficial plasma proteins or electrolytes. The test results obtained demonstrated that treatments using the adsorptive toxin removal device in conjunction with a commercially available kidney dialysis system, the Diapact^{™} continuous renal replacement therapy (CRRT) system (B|BRAUN Medical Inc., Bethlehem, PA) in plasma adsorption/perfusion (PAP) mode, were safe and well tolerated without detrimental hemodynamic effects or biocompatibility concerns.

Testing involved canine model extracorporeal circulation with the Diapact™ CRRT system in PAP mode was performed for a lead-in hour to determine the effects of the extracorporeal circuit without inclusion of plasma filtration and the HLM-100 adsorptive toxin removal device (blood loop). The plasma flow pump was then initiated with the adsorptive toxin removal device (adsorbent column) included into the plasma flow path of the extracorporeal circulation (plasma loop) for an additional four hours (total of 6 liters of plasma processed by adsorptive toxin removal device).

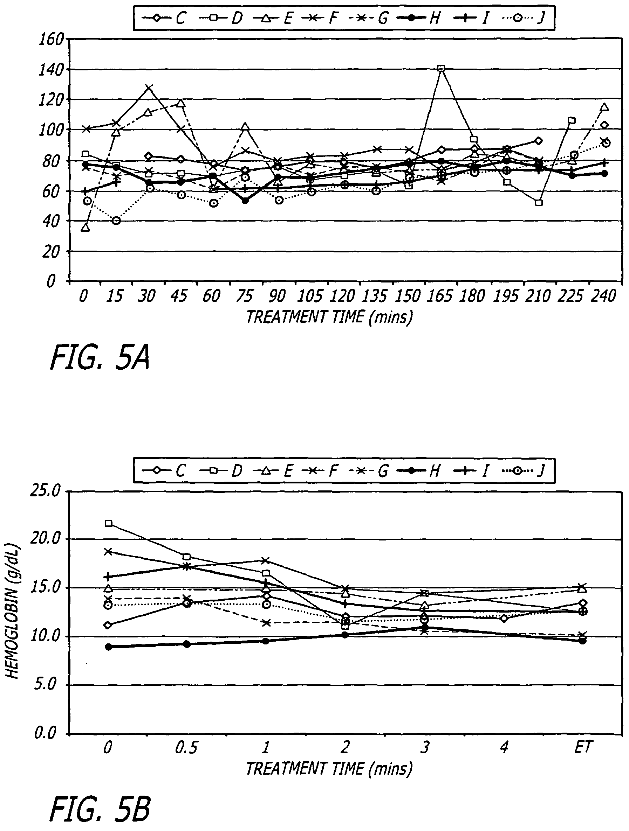

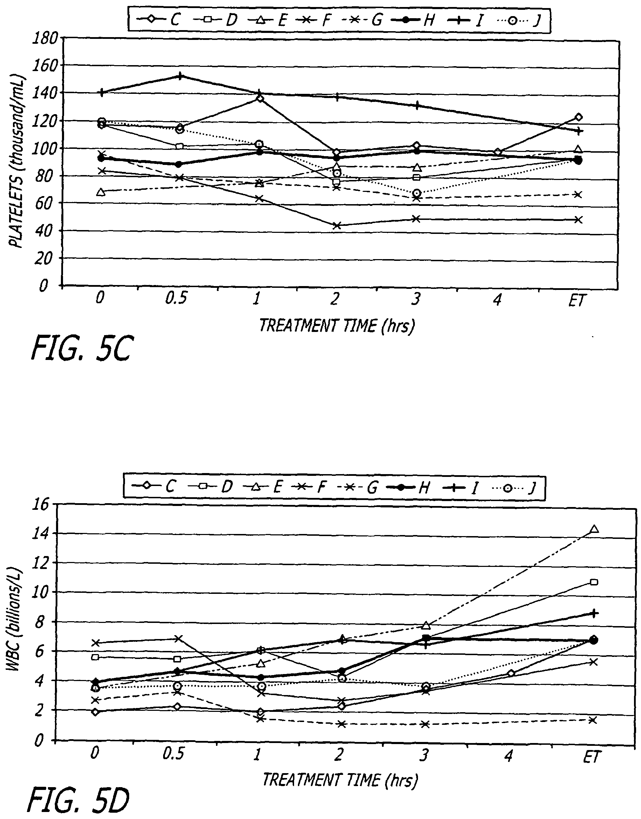

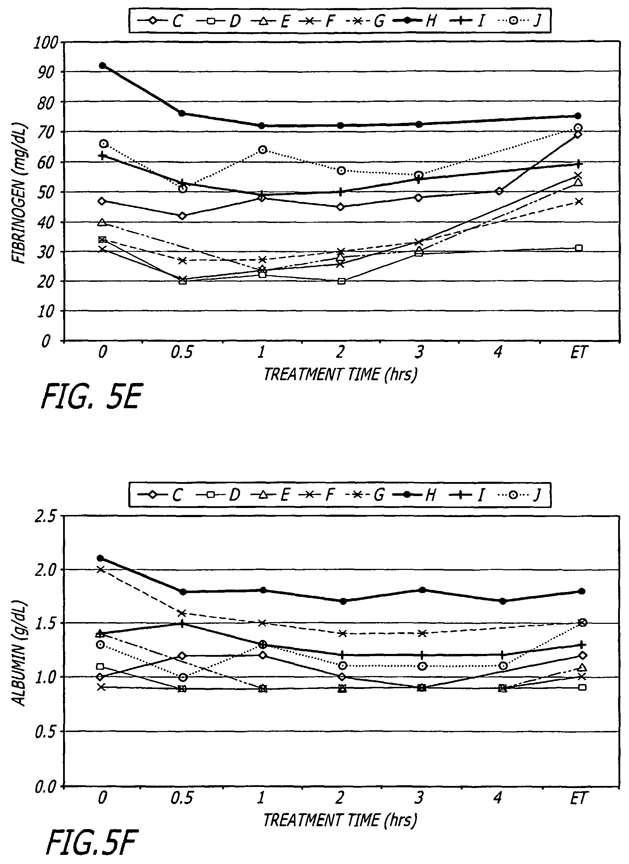

Inclusion of the HLM-100 adsorbent column into an extracorporeal circuit did not result in evidence of hemodynamic instability (FIG. 5a), hemolysis (FIG. 5b), thrombocytopenia (FIG. 5c), leucopenia (FIG. 5d), or nonspecific loss of fibrinogen (FIG. 5e) or albumin (FIG. 5f) (Animals identified as C-J; ET=End of Treatment values). Over the course of the four hours of extracorporeal circulation with the adsorptive column there was an increase in mean arterial pressure. Comparison of the various parameters pre- and post-inclusion of the HLM-100 adsorbent column confirmed that the inclusion of the adsorbent column was safe (see Table 2). There was also no evidence of adsorption column-related electrolyte abnormalities or consumption of clotting factors. There was minor anticoagulation-related bleeding noted at the cut down sites for the hemodialysis catheter in addition to the invasive hemodynamic monitoring catheters (pulmonary artery catheter and arterial catheter).

**Table 2: A comparison of the effect of the toxin removal device inclusion into an extracorporeal circuit in a canine model prior to and following conclusion of 4 hours of treatment.**

| | Prior to HLM-100 column inclusion in circuit | Following HLM-100 column inclusion in circuit for 4 hr | p value |
|---|---|---|---|
| Mean Arterial Pressure (mmHg) | 71±20 | 92±16 | <0.05 |
| Hemoglobin (g/dL) | 14.8±4.0 | 12.6±2.0 | NS |
| Platelet (Thousand/mL) | 104±23 | 93±24 | NS |
| WBC (Thousand/mL) | 4.0±1.5 | 7.9±3.8 | <0.05 |
| Serum Fibrinogen (mg/dL) | 52±22 | 58±14 | NS |
| Serum Albumin (g/dL) | 1.4±0.4 | 1.3±0.4 | NS |

There are significant advantages to this technique and technology over conventional renal dialysis. The use of an adsorption column allows for the targeted removal of both protein bound and soluble toxins associated with sepsis and renal failure that cannot be removed or removed efficiently, using standard dialysis techniques. In addition, the use of combined zero-balance ultrafiltration (ZBUF) and continuous veno-venous hemofiltration (CWH) facilitated the removal of waste materials and the stabilization of electrolytes as well as the simultaneous controlled removal of excess plasma water. And, the composition of the ZBUF replacement fluid can be customized to meet patient physiologic requirements.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements.

The terms "a" and "an" and "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g. "such as") provided herein is intended merely to better illuminate the invention and does not pose a limitation on the scope of the invention otherwise claimed. No language in the specification should be construed as indicating any non-claimed element essential to the practice of the invention.

Groupings of alternative elements or embodiments of the invention disclosed herein are not to be construed as limitations. Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is herein deemed to contain the group as modified thus fulfilling the written description of all Markush groups used in the appended claims.

Preferred embodiments of this invention are described herein, including the best mode known to the inventors for carrying out the invention. Of course, variations on those preferred embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Furthermore, numerous references have been made to patents and printed publications throughout this specification.

In closing, it is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

## Claims

1. An extracorporeal system for the removal of cytokines and toxins from plasma and to balance plasma water comprising:
a plasma filter (106) for separating plasma from blood;
an adsorption device (120) for removing cytokines and toxins from the plasma;
a hemofilter (128) for removing small molecules from the plasma and balancing plasma water;
wherein said adsorption device (120) comprises one or more adsorbents selected from the group consisting of activated carbon, non-ionic exchange resin and ion exchange resin,
**characterized in that** said adsorbents are coated with albumin prior to use in said adsorption device, and **in that** the hemofilter (128) combines zero-balance ultrafiltration and continuous veno-venous hemofiltration.

2. An extracorporeal system as claimed in claim 1 wherein said plasma filter (106) has a molecular weight cutoff greater than 0.45 microns.

3. An extracorporeal system as claimed in claim 1 wherein said activated carbon comprises uncoated coconut shell granule charcoal.

4. An extracorporeal system as claimed in claim 1 wherein said activated carbon comprises uncoated organic granule charcoal or uncoated synthetic carbon.

5. An extracorporeal system as claimed in claim 1 wherein said adsorbent is at least one ion exchange resin.

6. An extracorporeal system as claimed in claim 1 wherein said adsorbent is at least one non-ionic exchange resin.

7. An extracorporeal system as claimed in claim 6 wherein said at least one non-ionic exchange resin is selected from the group consisting of non-ionic aliphatic ester resins, non-ionic polystyrene divinyl benzene resins and other non-biologic adsorptive resins.

8. An extracorporeal system as claimed in claim 7 wherein at least one of said non-ionic aliphatic ester resins has an average surface area of approximately 500 m²/g and an average pore size of approximately 450 Angstroms and a mean particle diameter of 560 µm.

9. An extracorporeal system as claimed in claim 7 wherein at least one of said non-ionic polystyrene divinyl benzene resins has an average surface area of approximately 700 m²/g with an average pore size of 300 Angstroms and a mean particle diameter from 35 µm to 120 µm.

10. An extracorporeal system as claimed in claim. 7 wherein at least one of said non-ionic polystyrene divinyl benzene resins has an average surface area of approximately 600 m²/g with an average pore size of 300 Angstroms and a mean particle diameter from 300 µm to 500 film.

11. An extracorporeal system as claimed in claim 1 further comprising at least one particle filter (122) disposed within said extracorporeal system downstream of said adsorption device (120) and before cleansed plasma from the adsorption device is combined with blood from the plasma system.

12. An extracorporeal system as claimed in claim 1 wherein said human albumin coating consists essentially of albumin and a physiologic solution.

## Patentansprüche

1. Extrakorporales System zur Entfernung von Cytokinen und Toxinen aus Plasma und zum Bilanzieren des Plasmawassers, umfassend:
einen Plasmafilter (106) zum Abtrennen des Plasmas von Blut;
eine Adsorptionsvorrichtung (120) zum Entfernen von Cytokinen und Toxinen aus dem Plasma;
einen Hämofilter (128) zum Entfernen kleiner Moleküle aus dem Plasma und zum Bilanzieren des Plasmawassers;
wobei die Adsorptionsvorrichtung (120) ein oder mehr Adsorptionsmittel umfasst, die aus der Gruppe ausgewählt sind, bestehend aus Aktivkohle, nicht ionischem Austauscherharz und Ionenaustauscherharz,
**dadurch gekennzeichnet, dass** die Adsorptionsmittel vor der Verwendung in der Adsorptionsvorichtung mit Albumin beschichtet werden, und wobei der Hämofilter (128) die Nullabgleich-Ultrafiltration mit der kontinuierlichen venovenösen Hämofiltration kombiniert.

2. Extrakorporales System nach Anspruch 1, wobei der Plasmafilter (106) eine molekulare Ausschlussgrenze größer als 0,45 µm aufweist.

3. Extrakorporales System nach Anspruch 1, wobei die Aktivkohle eine nicht beschichtete Kornkohle aus Kokosnussschalen umfasst.

4. Extrakorporales System nach Anspruch 1, wobei die Aktivkohle nicht beschichtete organische Kornkohle oder nicht beschichteten synthetischen Kohlenstoff umfasst.

5. Extrakorporales System nach Anspruch 1, wobei das Adsorptionsmittel mindestens ein Ionenaustauscherharz ist.

6. Extrakorporales System nach Anspruch 1, wobei das Adsorptionsmittel mindestens ein nicht ionisches Austauscherharz ist.

7. Extrakorporales System nach Anspruch 6, wobei das mindestens nicht ionische Austauscherharz aus der Gruppe ausgewählt ist, bestehend aus nicht ionischen aliphatischen Esterharzen, nicht ionischen Polystyrol-Divinylbenzol-Harzen und anderen nicht biologischen Adsorptionsharzen.

8. Extrakorporales System nach Anspruch 7, wobei mindestens eines der nicht ionischen aliphatischen Esterharze einen durchschnittlichen Oberflächenbereich von ca. 500 m²/g und eine durchschnittliche Porengröße von ca. 450 Ängström und einen mittleren Partikeldurchmesser von 560 µm aufweist.

9. Extrakorporales System nach Anspruch 7, wobei mindestens eines der nicht ionischen Polystyrol-Divinylbenzol-Harze einen durchschnittlichen Oberflächenbereich von ca. 700 m²/g mit einer durchschnittlichen Porengröße von 300 Ängström und einem mittleren Partikeldurchmesser von 35 µm bis 120 µm aufweist.

10. Extrakorporales System nach Anspruch 7, wobei mindestens eines der nicht ionischen Polystyrol-Divinylbenzol-Harze einen durchschnittlichen Oberflächenbereich von ca. 600 m²/g mit einer durchschnittlichen Porengröße von 300 Ängström und einem mittleren Partikeldurchmesser von 300 µm bis 500 µm aufweist.

11. Extrakorporales System nach Anspruch 1, weiter umfassend mindestens einen Partikelfilter (122), der im extrakorporalen System nachgelagert von der Adsorptionsvorrichtung (120) und bevor das gereinigte Plasma aus der Adsorptionsvorrichtung mit Blut aus dem Plasmasystem kombiniert wird, angeordnet ist.

12. Extrakorporales System nach Anspruch 1, wobei die Beschichtung aus Humanalbumin im Wesentlichen aus Albumin und einer physiologischen Lösung besteht.

## Revendications

1. Système extracorporel destiné à enlever des cytokines et des toxines du plasma et à compenser l'eau plasmatique, comprenant :
un filtre à plasma (106) pour séparer le plasma du sang ;
un dispositif d'adsorption (120) pour enlever des cytokines et des toxines du plasma ;
un hémofiltre (128) pour enlever des petites molécules du plasma et pour compenser l'eau plasmatique ;
dans lequel ledit dispositif d'adsorption (120) comprend un ou plusieurs agents adsorbants choisis dans le groupe constitué par du charbon actif, une résine échangeuse non ionique et une résine échangeuse d'ions,
**caractérisé en ce que** lesdits agents adsorbants sont revêtus d'albumine avant d'être utilisés dans ledit dispositif d'adsorption et **en ce que** l'hémofiltre (128) combine une ultrafiltration à bilan nul et une hémofiltration continue veino-veineuse.

2. Système extracorporel selon la revendication 1, dans lequel ledit filtre à plasma (106) possède un seuil de masse moléculaire supérieur à 0,45 microns.

3. Système extracorporel selon la revendication 1, dans lequel ledit charbon actif comprend un charbon granulé non revêtu issu de coques de noix de coco.

4. Système extracorporel selon la revendication 1, dans lequel ledit charbon actif comprend du charbon granulé organique non revêtu ou un carbone synthétique non revêtu.

5. Système extracorporel selon la revendication 1, dans lequel ledit agent adsorbant est au moins une résine échangeuse d'ions.

6. Système extracorporel selon la revendication 1, dans lequel ledit agent adsorbant est au moins une résine échangeuse non ionique.

7. Système extracorporel selon la revendication 6, dans lequel ladite au moins une résine échangeuse non ionique est choisie dans le groupe constitué par des résines d'esters aliphatiques non ioniques, des résines de polystyrène et divinylbenzène non ioniques, et d'autres résines adsorbantes non biologiques.

8. Système extracorporel selon la revendication 7, dans lequel l'une au moins desdites résines d'esters aliphatiques non ioniques possède une aire de surface moyenne d'environ 500 m²/g avec une taille de pore moyenne d'environ 450 angströms et un diamètre de particule moyen de 560 µm.

9. Système extracorporel selon la revendication 7, dans lequel l'une au moins desdites résines de polystyrène et divinylbenzène non ioniques possède une aire de surface moyenne d'environ 700 m²/g avec une taille de pore moyenne de 300 angströms et un diamètre de particule moyen allant de 35 µm à 120 µm.

10. Système extracorporel selon la revendication 7, dans lequel l'une au moins desdites résines de polystyrène et divinylbenzène non ioniques possède une aire de surface moyenne d'environ 600 m²/g avec une taille de pore moyenne de 300 angströms et un diamètre de particule moyen allant de 300 µm à 500 µm.

11. Système extracorporel selon la revendication 1, comprenant en outre au moins un filtre à particules (122) disposé à l'intérieur dudit système extracorporel en aval dudit dispositif d'adsorption (120) et avant que le plasma épuré provenant du dispositif d'adsorption soit combiné avec du sang provenant du système plasmatique.

12. Système extracorporel selon la revendication 1, dans lequel ledit revêtement d'albumine humaine est constitué essentiellement d'albumine et d'une solution physiologique.
